# EUROPEAN PATENT APPLICATION

(11) **EP 0 692 296 A1**
(43) Date of publication of application: **17.01.1996**
(21) Application number: 94910544.9
(22) Date of filing: 28.03.1994
(51) Int. Cl.: B01D 53/14, B01D 53/34, C07C 215/18

(54) **CARBON DIOXIDE ABSORBENT**

(30) Priority: 30.03.1993 JP 71634/93
(71) Applicant: KYOWA HAKKO KOGYO CO., LTD., Chiyoda-ku, Tokyo 100 (JP); INTERNATIONAL CENTER FOR ENVIRONMENTAL TECHNOLOGY TRANSFER, Yokkaichi-shi, Mie 510-12 (JP)
(72) Inventor: ITO, Yukiyoshi, Tokyo 154 (JP); YAMANO, Junzo, Kawachinagano-shi, Osaka 586 (JP); NAGASATO, Kenji, Yokkaichi-shi, Mie 510 (JP)
(74) Representative: VOSSIUS & PARTNER
(86) International application number: JP9400492
(87) International publication number: WO9422560

(57) **Abstract**

A carbon dioxide absorbent containing a diamino alcohol represented by general formula (I) as the active ingredient, wherein R¹, R² and R³ may be the same or different from one another and each represents hydrogen or alkyl. This absorbent is excellent in that it can efficiently absorb carbon dioxide and efficiently desorb the absorbed gas event at a temperature of 100 °C or below.

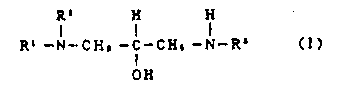

## Description

### FIELD OF THE INVENTION

The present invention relates to a carbon dioxide absorbent containing a diaminoalcohol as an active component.

### BACKGROUND OF THE INVENTION

Hydrochlorides of a part of diaminoalcohols, used in the present invention, represented by the formula (I): 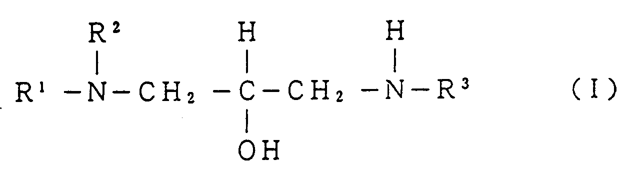
wherein R¹, R² and R³ are the same or different and represent hydrogen or alkyl, have been studied on the application to pharmaceutical preparations (J. Med. Chem., 23, 620 (1980); Chemical Abstracts (CA), Vol 50, 8742b (1956)). However, the above hydrochlorides are not known as a carbon dioxide absorbent.

There is known a carbon dioxide absorbent containing, as an active component, a diaminoalcohol (Compound A) represented by the formula (A): 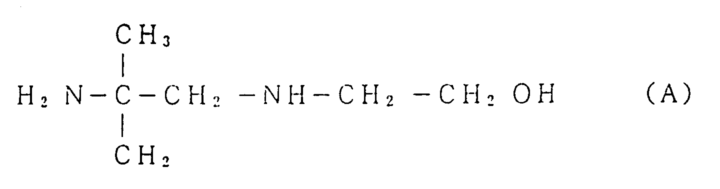
and also known that said absorbent desorbs carbon dioxide at 100 to 160 °C (USP 4,112,050).

There is known a carbon dioxide absorbent containing, as an active component, a diaminoalcohol (Compound B) represented by the formula (B): 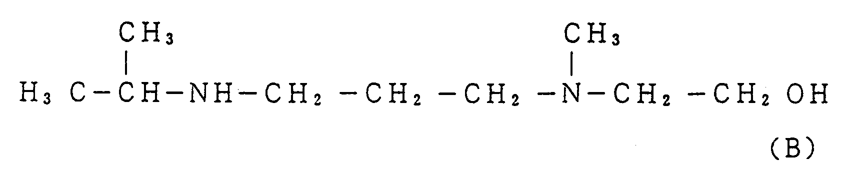
and also known that said absorbent desorbs carbon dioxide at 80 to 150 °C (JP-A 61-137845). Further, there is known a carbon dioxide absorbent containing, as an active component, an amine compound having a hydroxyl group and also known that said absorbent desorbs carbon dioxide at 80 to 150 °C (USP 4,112,052).

### OBJECT OF THE INVENTION

The object of the present invention is to provide a carbon dioxide absorbent which effectively absorbs carbon dioxide and effectively desorbs carbon dioxide even at a temperature of not higher than 100 °C.

### SUMMARY OF THE INVENTION

The present invention provides a carbon dioxide absorbent comprising, as an active component, a diaminoalcohol (Compound I) represented by the formula (I): 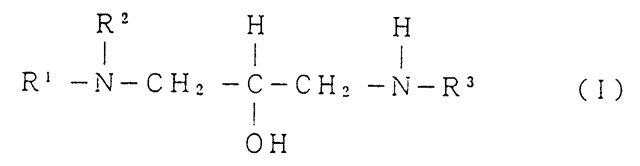
wherein R¹, R² and R³ are the same or different and represent hydrogen or alkyl, and a method for absorbing and desorbing carbon dioxide using the absorbent.

The carbon dioxide absorbent of the present invention has such the excellent properties that it effectively absorbs carbon dioxide and effectively desorbs even at a temperature of not higher than 100 °C.

### BRIEF DESCRIPTION OF DRAWING

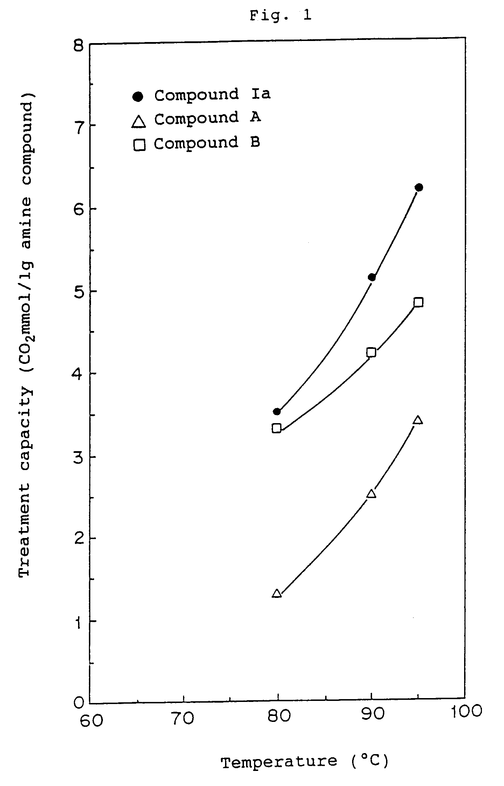

Figure 1 is a graph showing the carbon dioxide treatment capacity, per 1g amine compound, of the present carbon dioxide absorbent (Compound Ia) and, as a control, a solution of Compound A or Compound B in water.

### DETAILED DESCRIPTION OF THE INVENTION

In the definition of the formula (I), examples of alkyl are alkyl having 1 to 3 carbon atoms, for example, methyl, ethyl, n-propyl, isopropyl and the like.

Compound I is a known compound as described above and prepared by the known process (J. Med. Chem., 23, 620 (1980); Chemical Abstracts (CA), Vol 50, 8742b (1956)) or the similar process (JP-B 52-39829).

A representative example of Compound I is a diaminoalcohol (Compound Ia) represented by the formula (Ia): 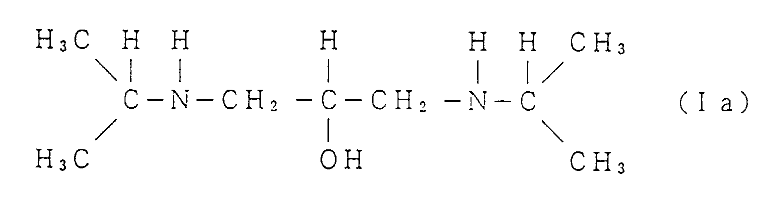
Next, a carbon dioxide absorbent containing Compound I as an active component is explained.

The carbon dioxide absorbent containing Compound I as an active component is contacted with, for example, a gas containing carbon dioxide at about 40 °C to absorb carbon dioxide in the absorbent. Then, the absorbed carbon dioxide is desorbed at 90 to 110 °C to recover carbon dioxide.

When Compound I is used as a carbon dioxide absorbing agent, it is normally diluted with water or water and a solvent.

For diluting Compound I with water, the concentration of Compound I is 10 to 90 wt%, preferably 15 to 50 wt%. When Compound I is used together with an inorganic compound such as alkali metal bicarbonate or carbonate or the like, the carbon dioxide absorbing properties of Compound I can be improved. In this case, the concentration of the inorganic compound is preferably 10 to 50 wt% and the concentration of Compound I is 2 to 20 wt%.

When Compound I is diluted water and a solvent, the concentration of Compound I is 10 to 90 wt%, preferably 15 to 50 wt%.

Examples of a solvent are N-alkylated pyrrolidone (N-methylpyrrolidone and the like), sulfone (sulfolane and the like), sulfoxide (dimethyl sulfoxide and the like), glycol (propylene glycol and the like), glycol ether (ethyl cellosolve and the like) and the like. The mixing ratio of the solvent with water is 5 to 95 wt%.

When a carbon dioxide absorbent (liquid) of the present invention is used industrially, for example, a gas containing carbon dioxide is contacted with the carbon dioxide absorbent in an absorbing tower to absorb carbon dioxide in the liquid carbon dioxide absorbent. Then, the liquid carbon dioxide absorbent having the absorbed carbon dioxide is heated at 90 to 110 °C in a regenerating tower to desorb carbon dioxide, to recover carbon dioxide as a gaseous component. A gas containing carbon dioxide is continuously supplied to the absorbing tower, while circulating again a liquid carbon dioxide absorbent from which carbon dioxide has been desorbed between the absorbing tower and the regenerating tower, to continuously desorb carbon dioxide from the regenerating tower, to recover carbon dioxide.

Thus, the carbon dioxide absorbent of the present invention is suitably used for absorbing carbon dioxide in the gases from a power plant, a factory and the like.

### EXAMPLE

The following Examples further illustrate the present invention in detail.

### Example 1

Each 50 ml of a solution of Compound Ia, A or B in water was separately placed in a stainless steel reactor having the content volume of 100 ml, and an inner temperature was adjusted to 40 °C while stirring at 800 rpm under the atmospheric pressure. A vent valve was closed, carbon dioxide was introduced into the reactor at the pressure of 1 kg/cm², through a conduit presubstituted with carbon dioxide, from a gas accumulator filled with carbon dioxide at about 25 kg/cm², and the introduced carbon dioxide was absorbed in the absorbent. The absorption completed within 10 to 15 minutes. The saturated absorption volume at an absorption temperature of 40 °C was obtained from the pressure drop in the gas accumulator.

The results are shown in Table 1.

**Table 1**

| Compound | Saturated absorption volume (CO₂ mmol/1g compound) |
|---|---|
| Ia | 8.05 |
| A | 5.60 |
| B | 6.95 |

Then, the vent valve was opened to remove the remaining gas in the reactor, an inner temperature in the reactor was raised at an elevating rate of 9 °C/min. (to 80°), 4 °C/min. (80 to 95 °C) under stirring, and the moisture generated simultaneously with carbon dioxide was removed by cooling and condensing. The generated amount of the resulting carbon dioxide was measured, and the desorbed ratio up to a temperature of 80, 90 or 95 °C (relative to the saturated absorption volume at 40 °C) was obtained. The results are shown in Table 2.

**Table 2**

| Compound | Desorption ratio (%) | | |
|---|---|---|---|
| | up to 80 °C | up to 90 °C | up to 95 °C |
| Ia | 44 | 64 | 77 |
| A | 24 | 44 | 60 |
| B | 48 | 60 | 69 |

Further, the amount of carbon dioxide desorbed at a temperature of 80, 90 or 95 °C from an absorbent solution saturated at 40 °C is shown in Figure 1, as the carbon dioxide (CO₂) treatment capacity per 1g amine (saturated absorption volume X desorption ratio). In Figure 1, ● represents the data of Compound Ia, △ represents those of Compound A, and □ represents those of Compound B.

From Figure 1, it is understood that Compound Ia has the considerably excellent CO₂ treatment capacity per unit (1g) in comparison with Compounds A and B at 90 and 95 °C. Therefore, it is clear that the carbon dioxide absorbent of the present invention containing Compound Ia as an active component is excellent over a carbon dioxide absorbent containing Compound A or B as an active component.

## Claims

1. A carbon dioxide absorbent comprising, as an active component, a diaminoalcohol represented by the formula (I): 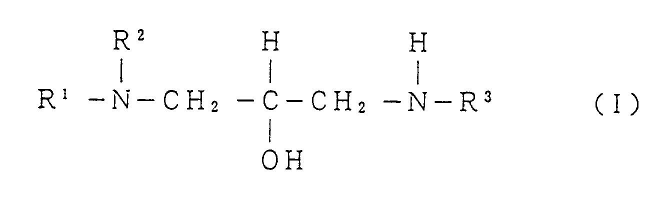 wherein R¹, R² and R³ are the same or different and represent hydrogen or alkyl.

2. The carbon dioxide absorbent according to claim 1, wherein the diaminoalcohol is represented by the formula Ia:

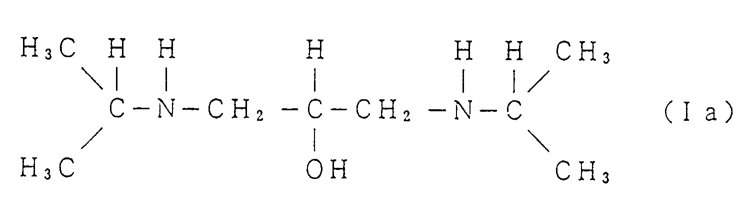

3. The carbon dioxide absorbent according to claim 1, the diaminoalcohol is diluted, with water, to the concentration of 10 to 90 wt%.

4. The carbon dioxide absorbent according to claim 1, wherein the diaminoalcohol was diluted, with water, to the concentration of 2 to 20 wt%, and alkali metal bicarbonate or carbonate was added therein at the concentration of 10 to 50 wt%.

5. The carbon dioxide absorbent according to claim 1, wherein the diaminoalcohol was diluted, with water, to the concentration of 10 to 90 wt%, wherein the mixing ratio of water and solvent is 5 to 95%, and the solvent is selected from the group consisting of N-alkylated pyrrolidone, sulfone, sulfoxide, glycol, glycol ether and mixture thereof.

6. A method for absorbing carbon dioxide which comprises contacting a carbon dioxide absorbent containing, as an active component, a diaminoalcohol represented by the formula (I): 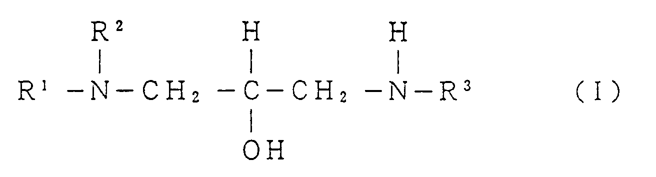 wherein R¹, R² and R³ are the same or different and are hydrogen or alkyl, with a gas containing carbon dioxide, to absorb carbon dioxide in the absorbent.

7. A method for desorbing carbon dioxide which comprises desorbing carbon dioxide which has been absorbed in the carbon dioxide absorbent according to claim 6, at a temperature of 90 to 110 °C.
